# EUROPEAN PATENT SPECIFICATION

(11) **EP 2 381 380 B1**
(45) Date of publication and mention of the grant of the patent: **31.07.2019**
(21) Application number: 11162730.3
(22) Date of filing: 15.04.2011
(51) Int. Cl.: G16H 10/00

(54) **Method and system for guiding clinicians in real time**
Verfahren und System zur Anleitung von Klinikern in Echtzeit
Procédé et système de guidage de praticiens en temps réel

(30) Priority: 23.04.2010 US 766334
(43) Date of publication of application: 26.10.2011
(73) Proprietor: General Electric Company, Schenectady, NY 12345 (US)
(72) Inventor: Mills, David Martin, Niskayuna NY 12309 (US); Dentinger, Aaron Mark, Niskayuna NY 12309 (US); Mackin, Michael Henry, Ellicott City MD 21042 (US)
(74) Representative: Fennell, Gareth Charles

(56) References cited:
- WO-A1-2009/129845
- JP-A- 2001 137 237
- US-A1- 2003 026 464
- US-A1- 2007 167 801
- US-B1- 6 458 081

## Description

### FIELD OF THE INVENTION

This invention relates generally to a clinician guidance system, and more particularly to, a method and system for guiding clinicians during clinical procedures by visually providing customized annotated images corresponding to each step in the procedure.

### BACKGROUND OF THE INVENTION

US 6 458 081 B1 discloses arranging activities of an ultrasonic diagnostic apparatus for realizing operations in accordance with the operation procedure.

Image guided clinical procedures are quite common and many times an inexperienced person may end up performing the clinical procedures due to various reasons. The clinician may need to be guided while performing a real-time imaging procedure and if the clinician is not an expert, it may be required to provide real time instructions to guide him. In an example, the clinician needs to be provided step-by-step instructions at various stages of the clinical procedure or the workflow. Further it might be difficult for an inexperienced person to simultaneously perform the procedure and adjust the imaging system to perform the desired procedure. With reference to a particular step, the clinician may need to adjust the settings of the imaging system and in many instances the clinician ends up performing the procedure using one hand and adjusting the imaging system using the other hand. Inexperienced people may fail to perform the same efficiently. Further, the clinical procedure is performed in a sterile environment and the controls of the imaging system or accessories may be located outside the sterile environment. So the person performing the clinical procedure are exposed to non-sterile environment and the patient safety is not assured adequately.

Considering the example of ultrasound systems, it is desirable to have a visually assisted image guidance system for inexperienced clinicians, and more particularly to a clinician performing an image guided procedure using an ultrasound imaging system. Ultrasound systems are configured to connect to an ultrasound probe to perform an ultrasound scan. Depending on the type of ultrasound scan to be performed, a probe having a particular transducer arrangement may be used that allows for performing the specific ultrasound scan. The ultrasound system usually includes a control portion that provides interfaces for receiving user inputs. For example, different buttons, knobs, etc. are provided to allow a user to select different options and control the scanning of an object using the connected ultrasound probe. Ultrasound guided interventional procedures are common in foliations such as breast biopsies, vascular access (e.g., placing of central line catheters), vein closure procedures and regional nerve blocks, among others. During these procedures, a clinician (or nurse) has to hold the ultrasound probe, in one hand and a needle in the other hand to perform the procedure, while observing the ultrasound image in real-time. However, in many cases, this type of procedure requires two individuals including an operator performing the scanning and another clinician performing the guidance procedure. Thus, using known ultrasound systems it is difficult to perform ultrasound imaging in combination with interventional procedures.

Further, in many instances clinical procedures using ultrasound imaging systems may not be performed by well-experienced clinicians or sonographers. Increased uptake of ultrasound in areas where people are not skilled in the area, require more assistance to understand the ultrasound images and to obtain relevant and clear images. In a specific example, inexperienced people may place the catheters through areas where they may take an incorrect turn or may become stuck. Clinical papers point to catheter misplacement as one of the most common causes of clinical complications that may lead to death. It will be beneficial to provide guidance to not so well experienced persons on catheter placement and this could reduce catheter misplacement and allow more frequent monitoring of these catheters. Further inexperienced people may find it difficult to place the catheter accurately, and may use more X-ray exposures to place and confirm the catheter position.

Therefore, it is desirable to provide a method and system for guiding clinicians during clinical procedures and for automating the configuration of the imaging system for performing various steps in the clinical procedure.

### SUMMARY OF THE INVENTION

The invention is set out in the accompanying independent claims. Advantageous embodiments are defined by the dependent claims.

### BRIEF DESCRIPTION OF THE DRAWINGS

**FIG. 1** is a block diagram of an ultrasound imaging system capable of guiding a clinician in real-time as described in an embodiment of the invention;
**FIG. 2** is a flowchart illustrating guiding a clinician using an image display device associated with an imaging system as described in an embodiment of the invention;
**FIG. 3** is a flowchart illustrating an ultrasound image guided procedure as described in an embodiment of the invention;
**FIG. 4** is a flowchart illustrating a visually assisted catheter guidance procedure as described in an embodiment of the invention;
**FIGs. 5A** - **5L** represent user interfaces showing various steps involved in a visually assisted catheter guidance procedure as described in an exemplary embodiment of the invention. Each of these sub-figures is further described as follows:

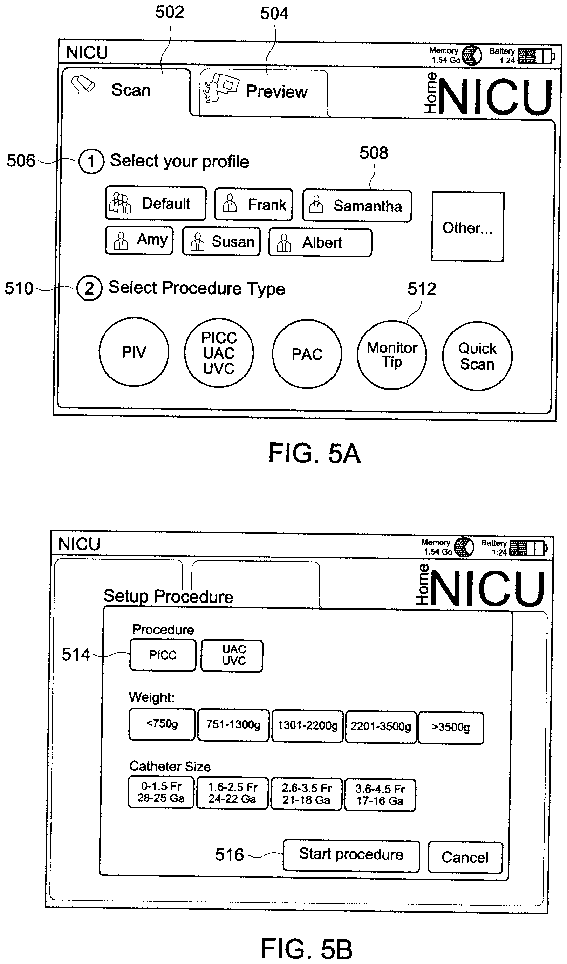

**FIG. 5A** represents a user interface configured for initiating a visually assisted catheter guidance procedure as described in an exemplary embodiment of the invention;
**FIG. 5B** represents a user interface configured for obtaining patient information in a visually assisted catheter guidance procedure as described in an exemplary embodiment of the invention;

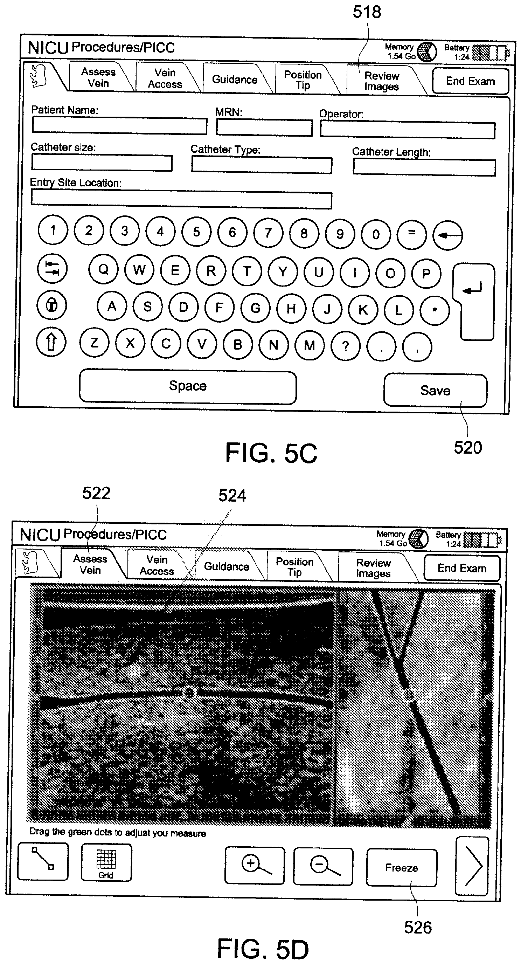

**FIG. 5C** represents a user interface configured for confirming patient and procedure information in a visually assisted catheter guidance procedure as described in an exemplary embodiment of the invention;
**FIG. 5D** represents a user interface configured for assessing vein in a visually assisted catheter guidance procedure as described in an exemplary embodiment of the invention;

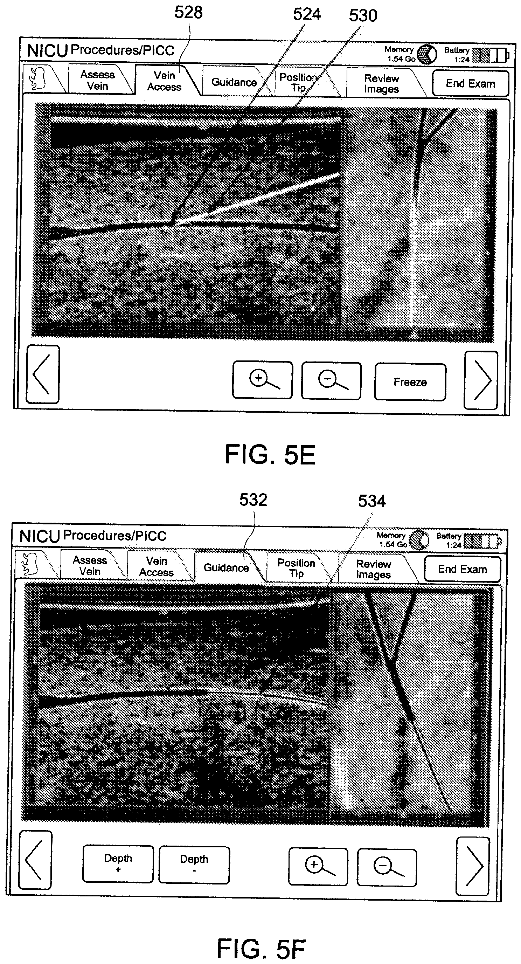

**FIG. 5E** represents a user interface configured for accessing the selected vein in a visually assisted catheter guidance procedure as described in an exemplary embodiment of the invention;
**FIG. 5F** represents a user interface configured for guiding catheter in the visually assisted catheter guidance procedure as described in an exemplary embodiment of the invention;

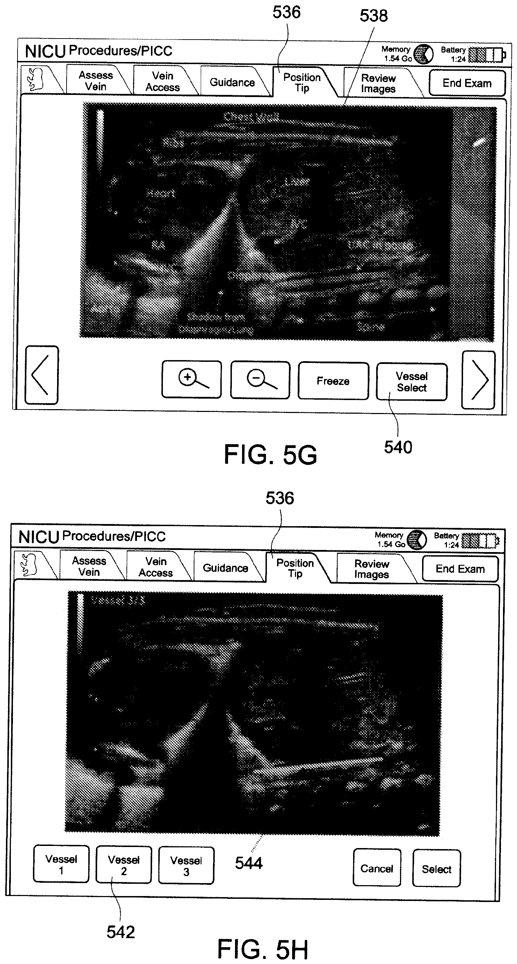

**FIG. 5G** represents a user interface configured for displaying annotated images in the visually assisted catheter guidance procedure as described in an exemplary embodiment of the invention;
**FIG. 5H** represents a user interface configured for selecting desired vessel in the visually assisted catheter guidance procedure as described in an exemplary embodiment of the invention;

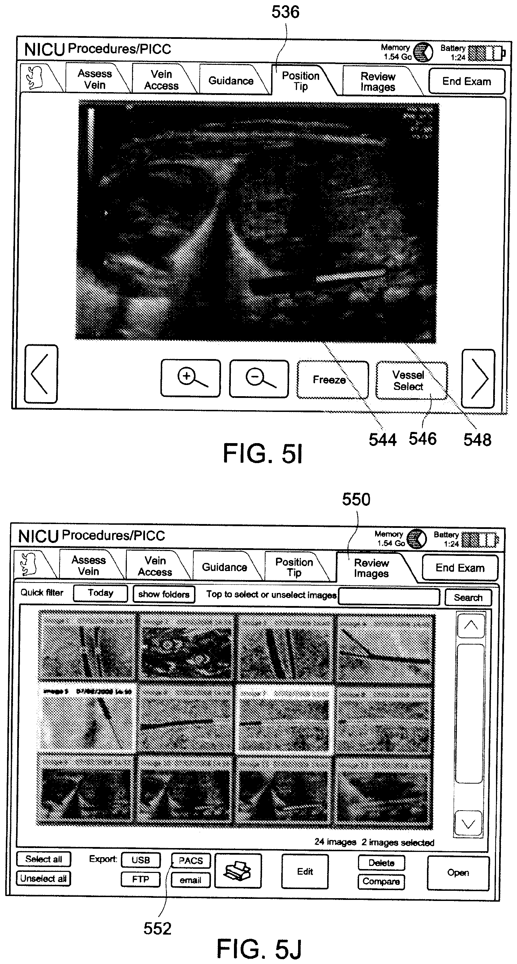

**FIG. 5I** represents a user interface configured for visually displaying the guidance in the selected vessel in a visually assisted catheter guidance procedure as described in an exemplary embodiment of the invention;
**FIG. 5J** represents a user interface configured for showing review of the guidance procedure and various utilities in a visually assisted catheter guidance procedure as described in an exemplary embodiment of the invention;

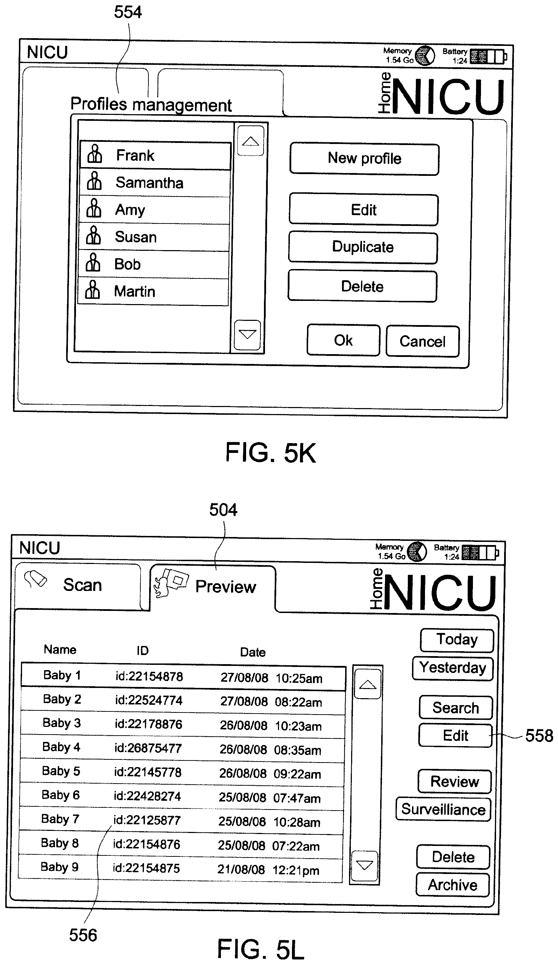

**FIG. 5K** represents a user interface configured for showing management process in a visually assisted catheter guidance procedure as described in an exemplary embodiment of the invention; and
**FIG. 5L** represents a user interface configured for showing review of various procedures in the visually assisted catheter guidance procedure as described in an exemplary embodiment of the invention;

### DETAILED DESCRIPTION OF THE INVENTION

In the following detailed description, reference is made to the accompanying drawings that form a part hereof, and in which is shown by way of illustration specific embodiments that may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken as limiting the scope of the invention. To the extent that the figures illustrate diagrams of the functional blocks of various embodiments, the functional blocks are not necessarily indicative of the division between hardware circuitry. Thus, for example, one or more of the functional blocks may be implemented in as single unit. Similarly, the programs may be stand alone programs, may be incorporated as subroutines in an operating system, may be functions in an installed software package, and the like. It should be understood that the various embodiments are not limited to the arrangements and instrumentality shown in the drawings.

In various embodiments, a method and system for guiding clinicians during clinical procedures by visually providing customized annotated images corresponding to each step in the procedure is disclosed. A desired workflow is selected by the clinician and each step in the workflow is displayed to the clinician, while the clinician performs the procedure. Further, based on the selected procedure, the imaging system is automatically configured to corresponding to each step in the workflow.

Embodiments of the present invention assist a clinician in a clinical procedure. Real-time assistance is provided by displaying annotated real time or sample images. Images corresponding to each step are selected and displayed to the clinician while performing that step.

In an exemplary embodiment, a visually assisted clinical guidance procedure is disclosed.

Another embodiment could include an imaging device to guide a biopsy procedure, such as a breast biopsy procedure. In such device, there would be an imaging system with some display device to guide a user to insert a needle into a specific area of interest. The imaging system could be used to assist the clinician to find and perhaps annotate an image and then guide a biopsy needle to the anatomy of interest. The image would be displayed in real-time with highlights in the area of interest and a workflow based upon that procedure. The first step of the procedure would be to locate the anatomy before needle guidance. Once the anatomy of interest is located and selected by the user, the clinician would advance to the next step where the clinician would be assisted in needle guidance to the previously selected anatomy.

Though the method is explained with reference to the ultrasound imaging system, the application of the invention need not be limited to ultrasound imaging systems. The method is applicable to any imaging modality and imaging operations performed for any anatomy. The device setting parameters are referred to parameters that controls the imaging operations and quality of the images, the parameters referred in the specification are few examples and need not be limited to these. Similarly, the catheter guidance procedure for central catheter procedures is for illustration purpose only, the invention could be applied in foliations such as breast biopsies, vascular access (e.g., placing of central line catheters), vein closure procedures and regional nerve blocks, among others. Further invention need not be limited to catheter guidance procedures, the method should assist clinician in placing various therapeutic device including RF tumor ablation probes, hemostatic probes, embolization catheters, electrophysiology catheters, endovascular catheters, biopsy needles, or any other location-critical therapies using electromagnetic navigation, however need not be limited to these. As used in this document, an annotated image is defined as an image that is at least labeled as to the appropriate step in the workflow and it may or may not contain labels and/or highlights on the current image and/or a sample image.

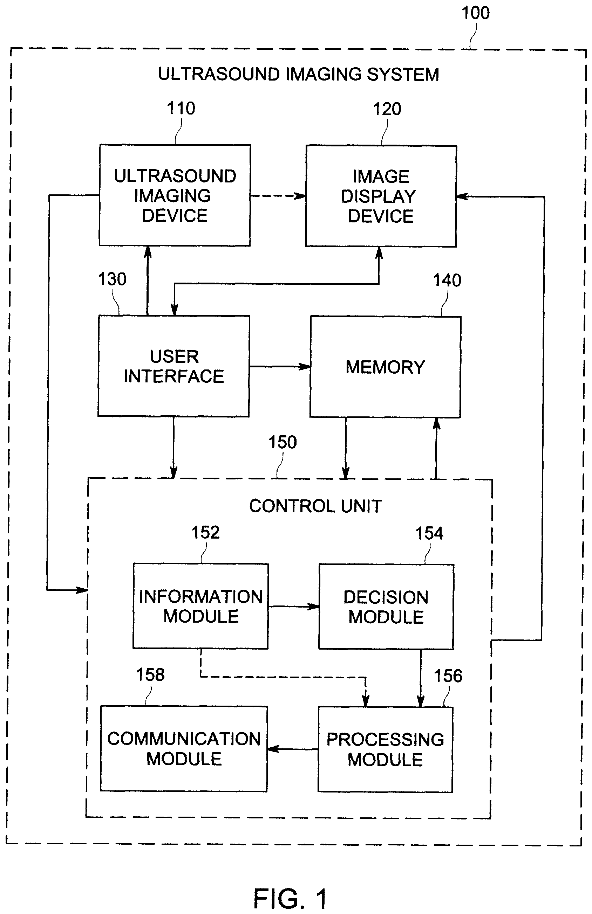

**FIG. 1** is a block diagram of an ultrasound imaging system capable of guiding a clinician in real time as described in an embodiment of the invention. The system 100 is configured to have an ultrasound imaging device 110 having a probe or transducer assembly (not shown) configured to acquire raw medical image data. The ultrasound imaging device 110 is associated with an image display device 120. The image display device 120 could be an image display associated with the imaging device 110 or an external image display. The image display device 120 is configured to display various medical images. The ultrasound imaging system 100 further comprises a user interface 130 configured to facilitate interaction between a clinician and the ultrasound imaging system 100 and also to facilitate interaction between the image display device 120 and the imaging system 100. The user interface 130 could be a visual interface that is displayed on the image display device 120 or any display associated with the imaging system 100. The user interface 130 may include, for example, a mouse, a touch screen and stylus, a keyboard with cursor keys, or combinations thereof. The user interface 130 may include a display as well. In an embodiment, the user interface 130 is a graphical user interface configured to interact with the clinician.

In an embodiment, the user interface 130 assists in obtaining patient information and the relevant clinical procedure. Various options for clinicians could be incorporated, if a graphical user interface is used. The user interface 130 could facilitate the clinician to create a user profile or access an existing user profile. Further, the clinician may also input the clinical procedure information such as type of procedure through the user interface 130.

Various clinical procedures, corresponding workflows, steps in the workflows, customized device setting parameters corresponding to each step in the workflow etc could be stored in a memory 140. The memory could be part of the ultrasound imaging system 100 or could be an external memory associated with the ultrasound imaging system 100. The memory 140 is stored/ archived with annotated images, either sample or real time images that could help clinician in performing the workflow. These predefined workflows and the annotated images corresponding to each clinical procedure provided by the experienced clinicians are stored in the memory 140. Alternately it could be stored in a server, which could be accessed based on the need. In an embodiment, along with the steps in the workflow, corresponding customized imaging parameters or any other device setting parameter may be stored in the memory 140. Alternately, a control unit 150 associated with the ultrasound imaging system 100 may derive the same in real time.

Once the user profile is created and stored in the memory 140, the user profile may be retrieved at a later stage. Further, the procedure, the images captured, analyzed etc may be stored corresponding to each patient and the same can be retrieved from the memory 140, if required. Further, the clinical procedures and images corresponding to various patients may be stored in the memory 140 and could be accessed as part of data management system.

The control unit 150 in the imaging system 100 is configured to process the images and assist clinicians in the real time procedure. The control unit comprises an information module 152, a decision module 154, a processing module 156 and a communication module 158. Dedicated hardware may be used instead of software and/or firmware for performing image processing, or a combination of dedicated hardware and software, or software in combination with a general purpose processor or a digital signal processor. Once the requirements for such software and/or hardware and/or dedicated hardware are gained from an understanding of the descriptions of embodiments of the invention contained herein, the choice of any particular implementation may be left to a hardware engineer and/or software engineer. However, any dedicated and/or special purpose hardware or special purpose processor is considered subsumed in the block labeled "control unit 150."

The information module 152 is configured to access various information from the memory 140. Initially, the patient information and relevant clinical procedure are accessed from the memory 140. Further, the information module 152 assists in accessing the workflows and the device setting parameters stored in the memory 140.

The decision module 154 is configured to select workflows based on the information provided by the information module 152. Once user profile and the relevant clinical procedure are identified, the clinician could identify the desired workflow and the decision module 154 selects desired workflow. The decision module 154 may select the workflow along with the steps involved in the workflow. In this event, the decision module may act as a user interface to receive the input on workflow selection or the selected workflow from the clinician. Alternately, the decision module 154 may assist clinician in identifying the desired workflow or automatically identify the workflow based on the patient information. The clinician may provide the details of the workflow, device specifics etc to the imaging system through the user interface 130. Once the workflow is identified or selected, the processing module 156 works with the information module 152 to access the details of the workflow including the steps involved in the workflow and the corresponding annotated images and the customized device setting parameters. The information module 152 may have the annotated sample images accessed from the memory 140 or the processing module 156 may annotate the same in real time based on the real time images. Further, the processing module 156 may derive the device setting parameters corresponding to each step in the workflow, if not available in the memory 140. The processing module 156 is further configured to receive the input from the user interface 130 and annotate the images based on the interaction with the clinician. The processing module 156 is further configured to configure the ultrasound imaging system 100 with the desired device setting parmaters during each step in the workflow. Further processing module 156 interacts with the user interface 130 to identify the current step of the workflow and assists the clinician by displaying the annotated images corresponding to the identified step. As and when the clinician passes through different steps in the workflow, the processing module 156 configures the imaging system 100 using the selected device setting parameters. Further processing module 156 could, annotate the images based on the clinician's interaction with the images or the workflow.

The communication module 158 is configured to communicate/display the workflow step by step along with the annotated image corresponding to each step. The communication module 158 is configured to identify the current step in the workflow being performed by the clinician from the processing module 156 and the annotated images corresponding to that particular step is displayed.

Thus the control unit 150 assists in automatically configuring the ultrasound imaging system 100 and the clinician gets real time assistance while performing the clinical procedure.

The processing module 156 may provide different utility controls such as printing the images, processing the images etc.

In an embodiment, the workflow is displayed with various tabs, indicating various steps in the workflow

In an embodiment, the user interface 130 is provided in a sterile environment. The user interaction with the system is kept as minimal and the user interface 130 could be pedal operated as the clinician can concentrate on the procedure. In an embodiment, the user interface 130 could be provided in association with the probe.

In an embodiment, the user interface 130 could be provided in the form of buttons on the probe, or a graphical interface on the imaging system 100 or on the image display device 120. Alternately, the user interface 130 could be provided in the form of buttons on the imaging system 100.

Embodiments of the present invention can comprise software or firmware instructing a computer to perform certain actions. Some embodiments of the present invention comprise stand-alone workstation computers that include memory, a display, and a processor along with the ultrasound imaging system. The workstation may also include a user input interface (which may include, for example, a mouse, a touch screen and stylus, a keyboard with cursor keys, or combinations thereof). The memory may include, for example, random access memory (RAM), flash memory, or read-only memory. For purposes of simplicity, devices that can read and/or write media on which computer programs are recorded are also included within the scope of the term "memory." A non-exhaustive list of media that can be read with such a suitable device includes CDs, CD-RWs, DVDs of all types, magnetic media (including floppy disks, tape, and hard drives), flash memory in the form of sticks, cards, and other forms, ROMs, etc., and combinations thereof.

Some embodiments of the present invention may be incorporated into a medical imaging apparatus, such as ultrasound imaging system 100 of Figure 1. In correspondence with a stand-alone workstation, the "computer" can be considered as the apparatus itself, or at least a portion of the components therein. For example, the control unit 150 may comprise a general purpose processor with memory, or a separate processor and/or memory may be provided. Image display device 120 corresponds to the display of the workstation, while the user interface 130 corresponds to the user interface of the workstation. Whether a stand-alone workstation or an imaging apparatus is used, software and/or firmware (hereinafter referred to generically as "software") can be used to instruct the computer to perform the inventive combination of actions described herein. Portions of the software may have specific functions, and these portions are herein referred to as "modules" or "software modules." However, in some embodiments, these modules may comprise one or more electronic hardware components or special-purpose hardware components that may be configured to perform the same purpose as the software module or to aid in the performance of the software module. Thus, a "module" may also refer to hardware or a combination of hardware and software performing a function.

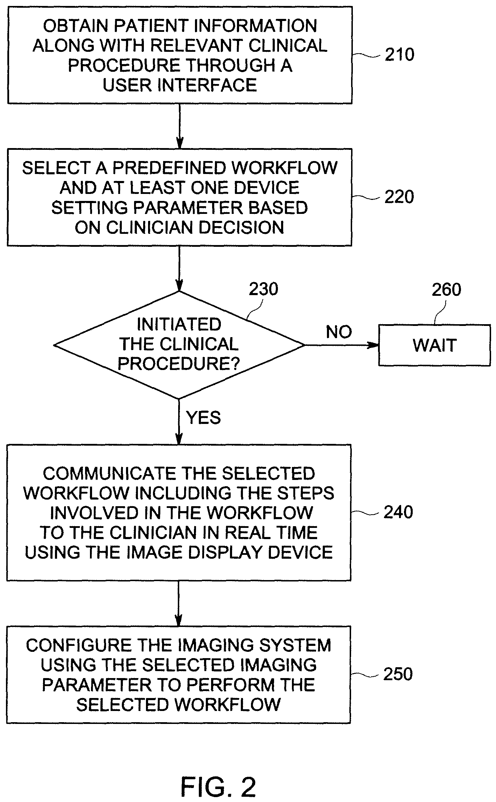

**FIG. 2** is a flowchart illustrating guiding a clinician using an image display device associated with an imaging system as described in an embodiment of the invention. At step 210, patient information along with the relevant clinical procedure is obtained using a user interface. The clinician suggests the relevant clinical procedure and the patient information could be obtained directly from the patient. Based on the patient information and the relevant clinical procedure, a predefined workflow and at least one device setting parameter may be selected from an information library, based on the clinician decision as at step 220. This workflow could be identified by the clinician and optionally the system could assist the clinician in selecting the workflow. The steps in the workflow or device setting parameters may or may not be selected using the patient information. Selecting the workflow could include selection of device setting parameters. Optionally the device setting parameters could be selected separately. An information library or a memory of the imaging system may be stored with various workflows corresponding to a clinical procedure and based on the patient information a desired workflow may be selected. Further, for each step in the workflow, the imaging system may have different device setting parameters, which may also be stored in the information library. The workflow includes various steps involved in the workflow along with the device setting parameters corresponding to each step in the workflow. A check is made at step 230, to confirm whether the clinical procedure has been initiated. The clinical procedure is initiated at the convenience of the clinician and the imaging system waits till the clinician initiates the procedure, as at step 260. Once the clinical procedure is initiated, the selected workflow is communicated to the clinician as at step 240. The workflow is communicated such that, as and when the clinician goes through each step in the workflow, corresponding step in the workflow is communicated to the clinician. The communicated workflow steps also include annotated images corresponding to each step along with the device setting parameters corresponding to each step. The annotated images could be displayed besides real time image or blended with the real time image. At step 250, the imaging system is configured with the selected device setting parameters. The device setting parameter is adjusted corresponding to each step in the workflow. As the clinician progresses through the workflow, corresponding step in the workflow is communicated along with the annotated images that could guide the clinician in performing that particular step. Further the imaging system is configured based on the device setting parameter corresponding to each step in the workflow, while the clinician performs a particular step. Device setting parameters could include frequency, focal depth, imaging depth etc. The imaging system may be configured to automatically or incorporating the suggestions from the clinicians.

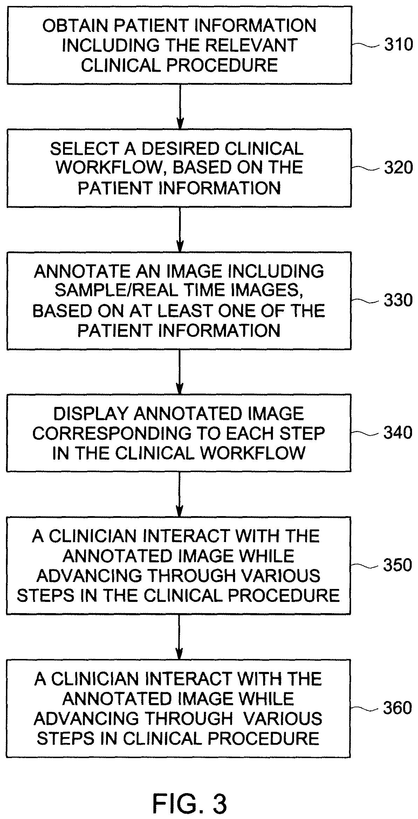

**FIG. 3** is a flowchart illustrating an ultrasound image guided procedure as described in an embodiment of the invention. The image guided procedure is performed with the help of an ultrasound imaging system associated with an image display device. At step 310, the patient information along with relevant clinical procedure information is obtained. This information is obtained prior to the ultrasound image guided procedure. At step 320, based on the patient information and the clinical procedure, a desired clinical workflow is selected. The desired workflow is identified by the clinician. A memory in the system could be stored with the different workflows, including the steps involved in the workflow and desired device setting parameters corresponding to each step in the workflow. At step 330, an image is annotated based on the patient information, clinical procedure and the current step in the workflow. Once the clinical procedure is initiated, workflow is communicated with annotated images, guiding the clinician to perform that particular step. The annotated image may include sample images or real time images. Annotating can be done in real time or the pre-annotated images could be accessed and displayed to the user as at step 340. Corresponding to each step in the workflow, annotated image is displayed. At step 350, the clinician interacts with the annotated images displayed and this helps the clinician in performing the clinical procedure. Once the clinician interacts with the images, further annotations could be done in real time based on the interaction, as at step 360.

For example, during a cardiac imaging operation, using ultrasound imaging system, the anatomy of heart may be annotated indicating with different parts of the heart and displayed to the clinician. This might help the clinician in identifying the area of interest. Once the area of interest is identified, different vessels in the area may be highlighted for the clinician. The clinician may interact with the images and may select a desired vessel of interest. The clinician could select the vessel and the annotated image may highlight the selected vessel. The annotated image may also guide the clinician to perform various procedures such as catheter guidance through the selected vessel. The annotation could be done on real time images or sample images. This procedure helps the clinician if they are unable to see a vessel or organ of interest inside the body.

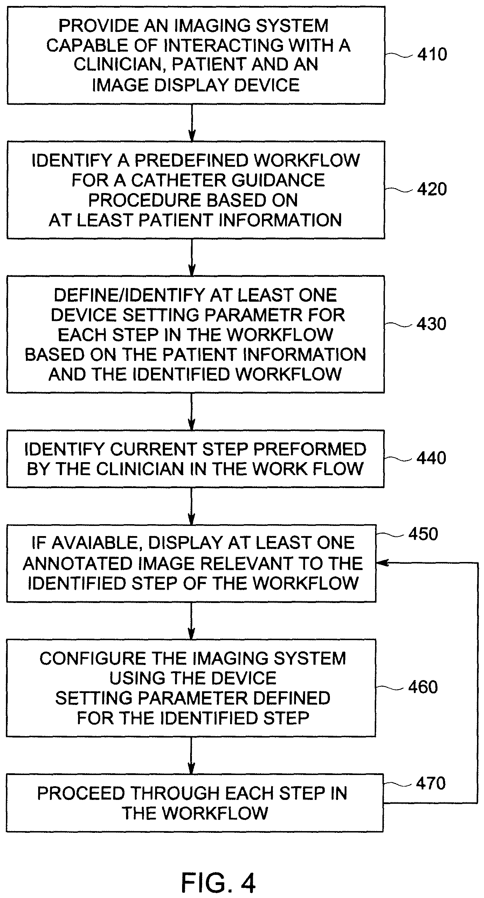

**FIG. 4** a flowchart illustrating a visually assisted catheter guidance process as described in an embodiment of the invention. At step 410, an imaging system capable of interacting with a clinician and an image display device is provided. The image display device could be display of the imaging system. At step 420, a predefined workflow for catheter guidance procedure is identified. The imaging system memory or any external memory may be stored with different workflows corresponding to various clinical procedures. The imaging is configured to interact with the clinician and obtains the patient information and relevant clinical procedure from the clinician. Once, the patient information is obtained, based on the patient information, the desired workflow is identified by the clinician. At step 430, at least one device setting parameter is identified/selected based on the patient information and/or the workflow. From patient details such as weight, age, and catheter size, the imaging system could identify certain customized device setting parameters such as depth, frequency etc for a particular step in the workflow. These parameters could be obtained as part of the patient information or could be derived based on the patient information or could be provided by the clinician. The clinical procedure and corresponding device setting parameters may vary with reference to different steps in the workflow. At step 440, the current step performed by the clinician is identified. The clinician may seek the guidance from the system from the beginning of the workflow or may interact with the system while the procedure is in progress. While the procedure is being performed, the clinician could interact with the system through user interface at various steps in the workflow. Interaction between the clinician and the system will allow the system to identify the current step in the procedure and the system could give the advice accordingly. At step 450, if available, at least one annotated image corresponding to the identified step in the workflow is displayed. The annotated images could be displayed with real time images. In case of a catheter guidance procedure, the system could show diagrams or pictures of how to position the probe in order to get high quality image and during a specific part of the insertion procedure. At step 460, the imaging system is configured using the selected device setting parameter. The system varies the present imaging system configuration based upon the expected imaging situation. For example, as and when the vein is deeper into the tissue, the frequency of the imaging needs to be adjusted. Based on the stages in the workflow, these device setting parameters are identified, and before each step, the imaging system is configured with the corresponding device setting parameter. At step 470, the clinical procedure is performed by proceeding through various steps in the workflow. For each step, the annotated images are displayed to guide the clinician and the imaging system is configured with the defined device setting parameters. The visually assisted catheter guidance procedure is helpful to provide guidance for catheter insertion procedure in the neonatal intensive care unit (NICU).

**FIG. 5A- 5F** represent various steps involved in a visually assisted catheter guidance procedure as described in an exemplary embodiment of the invention. The features shown are exemplary and various other features relating to image processing/storage or guidance or imaging could be provided. **FIG. 5A** represents a user interface configured for initiating the visually assisted catheter guidance procedure as described in an exemplary embodiment of the invention. The user interface includes two interfaces namely a Scan Interface 502 and a Review interface 504. The Scan Interface 502 assists the clinician in catheter guidance procedure and the Review Interface 504 assist the clinician in data management. The Scan Interface 502 visually communicates the need for user profile and the clinical procedure information. The user profile could be saved, in the system or in a server and could be assessed before the procedure. The Scan Interface 502 provides Patient Information Interface 506 to provide the patient information and a Procedure Information Interface 512 to provide the clinical procedure information. Available user profiles 508 could be displayed or the user may provide patient identification details such as patient name or hospital Identification information and the user profile may be accessed or created. The desired clinical procedure could be identified by the clinician and the same has to be selected using the procedure Information Interface 510. A drop down menu with available procedure may be provided as the Procedure Information Interface 510 and could be accessed by the clinician. Alternately different options could be displayed as the Procedure Information Interface 510. Corresponding to each procedure available, predefined set of steps or workflows have been defined by clinicians. Addition Interfaces 512 are provided to assist the clinician in performing a quick scan or understanding the display device. The user interface shown in FIG. 5A is used for initiating the visually assisted catheter guidance procedure.

**FIG. 5B** represents a user interface configured for obtaining procedure information in a visually assisted catheter guidance procedure as described in an exemplary embodiment of the invention. Once the desired clinical procedure is selected by the Procedure Information Interface 512, the details about procedure are obtained using Detail Information Interface 514. Few examples of the details include procedure type, catheter parameters etc. Various functions in Detail Information Interface 514 could be created based on the application and details of the procedure could be obtained. It is to be noted that the example need not be limited to the ones shown. Once the procedure details are obtained through the Detail Information Interface 514, the clinician may start with the procedure by accessing a Start Procedure Interface 516.

**FIG. 5C** represents a user interface configured for confirming patient and procedure information in a visually assisted catheter guidance procedure as described in an exemplary embodiment of the invention. This interface helps in confirming the patient and procedure information. This helps in improving the safety of the procedure and the features provided are exemplary and need not be limited to the described ones. The workflow is displayed with tabs 518 showing different steps in the procedure. The tab indicating the current step will be shown highlighted. A Save Interface 520 is provided to save the patient information along with the procedure information. Once the information is saved, it is assumed that the information is verified by the clinician or the user.

**FIG. 5D** represents a user interface configured for assessing vein in the visually assisted catheter guidance procedure as described in an exemplary embodiment of the invention. Once the details are confirmed, the systems assess the area of interest, as indicated by the Assess Interface 522. In the example shown, the clinician assesses a vein 524. To assist the clinician in assessing the vein 524, a Freeze Interface 526 is provided. The Freeze Interface 526 will help the clinician in assessing the vein 524 using linear measure or Grid method. Theses methods are exemplary and various functions could be defined to assist the clinician in assessing the area of interest. The vein 524 could be assessed, in view of the user profile and identified procedure.

**FIG. 5E** represents a user interface configured for accessing the selected vein in a visually assisted catheter guidance procedure as described in an exemplary embodiment of the invention. Once the vein 524 is identified and assessed, an Access Interface 528 will assist the clinician in accessing the vein 524 or the area of interest in the vein 524. The area of interest in the vein 524 is accessed by a needle 530 as shown in the image. This helps the clinician in accessing the vein 524.

**FIG. 5F** represents a user interface configured for guiding a catheter in the visually assisted catheter guidance procedure as described in an exemplary embodiment of the invention. A Guidance Interface 532 is provided to guide the clinician in placing a catheter 534. The catheter 534 is shown in the Guidance Interface 532 as being inserted into the areas of interest of the selected vein 524. Different user controls such as zoom, depth etc could be provided to control the display. This interface helps the clinician by showing the clinician how the catheter 534 needs to be inserted into and fed through the vein 524.

**FIGs. 5G, 5H****, and** **5I** represent a user interface configured for assisting a clinician in positioning a catheter. **FIG. 5G** represents a user interface configured for displaying annotated image in the visually assisted catheter guidance procedure as described in an exemplary embodiment of the invention. A Position Tip Interface 536 is provided to assist a clinician in positioning the catheter 534. Real time image or sample images of an interested anatomy are displayed with different parts of the anatomy being identified. The annotated image 538 of the anatomy is displayed in **FIG.5G****.** This will help the clinician to understand the anatomy better. The Position Tip Interface 536 is provided with Vessel Select Interface 540, capable of assisting a clinician in selecting the vessel of interest. Once the Vessel Select Interface 540 is clicked different vessels in the areas of interest is displayed to the user as in **FIG. 5H****.**

**FIG. 5H** represents a user interface configured for selecting desired vessel in the visually assisted catheter guidance procedure as described in an exemplary embodiment of the invention. The Position Tip Interface 536 provides plurality of Vessel Select Option Interface 542 to select the desired vessel of interest. Once a vessel is selected, the selected vessel 544 is highlighted and displayed.

**FIG. 5I** represents a user interface configured for visually displaying the guidance in the selected vessel in a visually assisted catheter guidance procedure as described in an exemplary embodiment of the invention. A Select Interface 546 is provided for selecting the catheter guidance procedure in the selected vessel. Once Select Interface 546 is clicked, a catheter guidance display 548 is shown through the selected vessel 544. Different control options could be provided to zoom and pause the guidance display.

**FIG. 5J** represents a user interface configured to show the review of the procedure and various utilities in the visually assisted catheter guidance procedure as described in an exemplary embodiment of the invention. A Review Image Interface 550 helps the clinician in reviewing the images. The Review Image Interface 550 includes different Utility Control Interfaces 552 such as edit, delete, compare, store etc.

**FIG. 5K** represents an initiation user interface for management process in the visually assisted catheter guidance procedure as described in an exemplary embodiment of the invention. A Profile Management Interface 554 is provided for creating and managing user profiles.

**FIG. 5L** represents a user interface configured to show the review of various patients and procedures in the visually assisted catheter guidance procedure as described in an exemplary embodiment of the invention. The Review Image Interface 504 displays scan information 556 related to various patients and helps in the data management. Further a Control Interface 558 is provided to manage the patient data.

In yet other embodiments of the present invention, a machine readable medium or media may include, but not limited to, magnetic disks and diskettes, optical disks and diskettes, and/or ROM, flash ROM, and/or battery backed RAM, or any other suitable magnetic, optical, or electronic medium or media) is provided. The medium (or media) has recorded thereon instructions configured to instruct a system 100 that includes an imaging device 110, image display device 120, user interface 130, memory 140, control unit 150 as shown in FIG.1, for guiding a clinician. The instructions includes: instructions for obtaining patient information along with relevant clinical procedure through a user interface; and selecting a predefined workflow and at least one device setting parameter from an information library, based on patient information and/or nature of workflow. The media further comprises: instructions for communicating the selected workflow including the steps in the workflow to the clinician in real time using the image display system, upon initiating the clinical procedure; and instructions for configuring the imaging system using the selected device setting parameter to perform the selected workflow.

In yet another embodiment, the medium (or media) has recorded thereon instructions configured to instruct a system 100 that includes an imaging device 110, image display device 120, user interface 130, memory 140, control unit 150 as shown in FIG.1, for an ultrasound image guided procedure. The instructions include: instructions for obtaining patient information including the relevant clinical procedure and for selecting a desired clinical workflow, based on the clinician decision. The media further includes: instructions for annotating an image based on at least one of the patient information; displaying annotated image, corresponding to each step in the clinical workflow, at least besides and/or blended into the real time image; and for interacting a clinician with the annotated image while advancing through various steps in the clinical procedure.

In yet another embodiment, the medium (or media) has recorded thereon instructions configured to instruct a system 100 that includes an imaging device 110, image display device 120, user interface 130, memory 140, control unit 150 as shown in FIG.1, for a visually assisted catheter guidance procedure. The instructions include: instructions for providing an imaging system capable of interacting with a clinician, patient and an image display device; identifying a predefined workflow for a catheter guidance procedure based on the clinician decision; defining at least one device setting parameter for each step in the workflow based on the patient information and the identified workflow; identifying current step preformed by the clinician in the work flow; displaying at least one annotated image relevant to the identified step of the workflow; and configuring the imaging system using the device setting parameter defined for the identified step.

The advantages of the invention include providing assistance to understand the ultrasound images and catheter guidance procedure for the clinicians. The real time guidance improves the chances of successful placement of catheters and thereby reduction in the number of x-rays used to confirm catheter placement, thus improved health of the patient. It may enable increased success for procedures out at hospital beds and a decreased need for surgical and other more stressful intervention.

The above-description of the embodiments of the methods and systems has the technical effect of guiding clinicians in a real time imaging procedure.

Thus various embodiments of the invention describe a method and system for guiding clinicians during clinical procedures by visually providing customized annotated images corresponding to each step in the procedure.

As used herein, an element or step recited in the singular and proceeded with the word "a" or "an" should be understood as not excluding plural elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising" or "having" an element or a plurality of elements having a particular property may include additional such elements not having that property.

Exemplary embodiments are described above in detail. The assemblies and methods are not limited to the specific embodiments described herein, but rather, components of each assembly and/or method may be utilized independently and separately from other components described herein. Further the steps involved in the workflow need not follow the sequence in which there are illustrated in figures and all the steps in the work flow need not be performed necessarily to complete the method.

While the invention has been described with reference to preferred embodiments, those skilled in the art will appreciate that certain substitutions, alterations and omissions may be made to the embodiments without departing from the scope of the invention. Accordingly, the foregoing description is meant to be exemplary only, and should not limit the scope of the invention as set forth in the following claims.

## Claims

1. A method of guiding a clinician performed by an ultrasound imaging system having a user interface, a control unit, a memory and an image display device comprising:
obtaining patient information (210) along with a relevant clinical procedure through a user interface;
selecting a predefined workflow and at least one device setting parameter (220) from an information library, based on a clinician decision obtained through user interface;
communicating the selected workflow (240) including the steps in the workflow to the clinician in real time using the image display device, upon initiating the clinical procedure; and
configuring the imaging system (250) using the selected device setting parameter to perform the selected workflow;
wherein communicating the selected workflow further comprises:
annotating an image (330) based on at least one of the patient information and steps in the workflow, the image including sample images and real time images of the patient;
displaying annotated image (340), corresponding to each step in the clinical workflow, at least one of besides and blended into the real time image; and
interacting a clinician with the annotated image (350).

2. The method as claimed in claim 1, wherein the information library (230) has annotated sample images and real time patient images annotated based on the patient information and predefined workflows.

3. The method as claimed in any preceding claim, wherein the step of communicating pre-defined workflow (240) includes displaying the workflow step by step as the clinician proceed through various steps in the procedure.

4. The method as claimed in any preceding claim, wherein each step in the workflow is displayed with corresponding annotated images, annotated images being displayed besides or blended with the images.

5. The method as claimed in claim 1, wherein the method further comprises:
identifying at least one device setting parameter based on patient information and the selected workflow, the device setting parameter being identified corresponding to each step in the workflow.

6. The method as claimed in claim 5, wherein the method comprises: presetting device setting parameters of the imaging system using the identified image setting parameters, the image setting parameters being adjusted during each step in the workflow.

7. The method as claimed in any one of claims 1 to 6, wherein the method further comprises: automatically configuring the ultrasound imaging system while the clinician advances through the steps in the workflow.

8. The method of any preceding claim, wherein the selected predefined workflow corresponds to the clinical procedure such that imaging system device setting parameters are selected.

9. An ultrasound imaging system (100) comprising:
an ultrasound imaging device (110) having a probe with a transducer assembly;
an image display device (120) associated with the imaging device configured to visually communicate with a clinician;
a memory (140) configured to store various pre-defined workflows and annotated images corresponding to various steps in the workflow;
a user interface (130) configured to facilitate interaction of the clinician with the imaging device (110) and interaction between the imaging device (110) and the display device (120) so that a predefined workflow is selectable; and
a control unit (150) configured to guide the clinician by displaying steps in the workflow along with the annotated images corresponding to each step, the annotated images being based on at least one of the patient information and steps in the workflow, the image including sample images and real time images of the patient;
wherein the control unit (150) comprises:
an information module (152) configured to access the workflows from the memory and the patient information;
a decision module (154) for selecting a desired workflow based on at least a clinician decision obtained through the user interface and device setting parameters corresponding to each step in the workflow;
a processing module (156) for processing images and configuring the ultrasound imaging device (110) with selected device setting parameter; and
a communication module (158) for communicating the annotated images by displaying the annotated images, corresponding to each step in the clinical workflow, at least one of besides and blended into the real time image; and interacting a clinician with the annotated image (350) while the clinician proceeds through these steps .

10. The system as claimed in claim 9, wherein the control unit (150) is configured to allow the clinician to select a workflow based on at least patient information and clinical procedure.

11. The system of claim 9 or claim 10, wherein the selected predefined workflow based on the clinical decision enables imaging device (110) setting parameters to be selected.

## Patentansprüche

1. Verfahren zur Anleitung eines Klinikers, das von einem Ultraschallbildgebungssystem durchgeführt wird, das eine Benutzerschnittstelle, eine Steuerungseinheit, einen Speicher und eine Bildanzeigeeinrichtung aufweist, umfassend:
Erhalten von Patienteninformationen (210) zusammen mit einem relevanten klinischen Verfahren über eine Benutzerschnittstelle;
Auswählen eines vordefinierten Arbeitsablaufs und mindestens eines Einrichtungseinstellparameters (220) aus einer Informationsbibliothek basierend auf einer Entscheidung eines Klinikers über die Benutzerschnittstelle;
Kommunizieren des ausgewählten Arbeitsablaufs (240), der die Schritte in dem Arbeitsablauf umfasst, in Echtzeit an den Kliniker mithilfe der Bildanzeigeeinrichtung nach Beginn des klinischen Verfahrens; und
Konfigurieren des Bildgebungssystems (250) mithilfe des ausgewählten Einrichtungseinstellparameters, um den ausgewählten Arbeitsablauf durchzuführen;
wobei das Kommunizieren des ausgewählten Arbeitsablaufs ferner umfasst:
Versehen eines Bildes mit Anmerkungen (330) basierend auf mindestens einem von den Patienteninformationen und den Schritten in dem Arbeitsablauf, wobei das Bild Beispielbilder und Echtzeitbilder des Patienten umfasst;
Anzeigen des mit Anmerkungen versehenen Bildes (340), das jedem Schritt in dem klinischen Arbeitsablauf entspricht, mindestens entweder neben dem Echtzeitbild oder damit verschmolzen; und
Interagieren eines Klinikers mit dem mit Anmerkungen versehenen Bild (350).

2. Verfahren nach Anspruch 1, wobei die Informationsbibliothek (230) mit Anmerkungen versehene Beispielbilder und Echtzeitpatientenbilder umfasst, die basierend auf den Patienteninformationen und vordefinierten Arbeitsabläufen mit Anmerkungen versehen sind.

3. Verfahren nach einem vorhergehenden Anspruch, wobei der Schritt des Kommunizierens des vordefinierten Arbeitsablaufs (240) das Schritt-für-Schritt-Anzeigen des Arbeitsablaufs umfasst, wenn der Kliniker verschiedene Schritte in dem Verfahren durchläuft.

4. Verfahren nach einem vorhergehenden Anspruch, wobei jeder Schritt in dem Arbeitsablauf mit entsprechenden mit Anmerkungen versehenen Bildern angezeigt wird, wobei die mit Anmerkungen versehenen Bilder neben den Bildern oder damit verschmolzen angezeigt werden.

5. Verfahren nach Anspruch 1, wobei das Verfahren ferner umfasst: Identifizieren von mindestens einem Einrichtungseinstellparameter basierend auf den Patienteninformationen und dem ausgewählten Arbeitsablauf, wobei der Einrichtungseinstellparameter entsprechend jedem Schritt in dem Arbeitsablauf identifiziert wird.

6. Verfahren nach Anspruch 5, wobei das Verfahren umfasst: Voreinstellen der Einrichtungseinstellparameter des Bildgebungssystems mithilfe der identifizierten Bildeinstellparameter, wobei die Bildeinstellparameter während jedes Schritts in dem Arbeitsablauf angepasst werden.

7. Verfahren nach einem der Ansprüche 1 bis 6, wobei das Verfahren ferner umfasst: automatisches Konfigurieren des Ultraschallbildgebungssystems, während der Kliniker die Schritte in dem Arbeitsablauf durchläuft.

8. Verfahren nach einem vorhergehenden Anspruch, wobei der ausgewählte vordefinierte Arbeitsablauf dem klinischen Verfahren entspricht, sodass die Bildgebungssystemeinrichtungseinstellparameter ausgewählt werden.

9. Ultraschallbildgebungssystem (100) umfassend:
eine Ultraschallbildgebungseinrichtung (110), die eine Sonde mit einer Wandleranordnung aufweist;
eine Bildanzeigeeinrichtung (120), die mit der Bildgebungseinrichtung verknüpft und dazu ausgelegt ist, visuell mit einem Kliniker zu kommunizieren;
einen Speicher (140), der dazu ausgelegt ist, verschiedene vordefinierte Arbeitsabläufe und mit Anmerkungen versehene Bilder, die verschiedenen Schritten in dem Arbeitsablauf entsprechen, zu speichern;
eine Benutzerschnittstelle (130), die dazu ausgelegt ist, die Interaktion des Klinikers mit der Bildgebungseinrichtung (110) und die Interaktion zwischen der Bildgebungseinrichtung (110) und der Anzeigeeinrichtung (120) zu erleichtern, sodass ein vordefinierter Arbeitsablauf auswählbar ist; und
eine Steuerungseinheit (150), die dazu ausgelegt ist, den Kliniker durch Anzeigen der Schritte in dem Arbeitsablauf neben den mit Anmerkungen versehenen Bildern, die jedem Schritt entsprechen, anzuleiten, wobei die mit Anmerkungen versehenen Bilder auf mindestens einem von den Patienteninformationen und den Schritten in dem Arbeitsablauf basiert sind, wobei das Bild Beispielbilder und Echtzeitbilder des Patienten umfasst;
wobei die Steuerungseinheit (150) umfasst:
ein Informationsmodul (152), das dazu ausgelegt ist, aus dem Speicher auf die Arbeitsabläufe und auf die Patienteninformationen zuzugreifen;
ein Entscheidungsmodul (154) zum Auswählen eines gewünschten Arbeitsablaufs basierend auf mindestens einer Entscheidung des Klinikers, die über die Benutzerschnittstelle erhalten wurde, und Einrichtungseinstellparametern, die jedem Schritt in dem Arbeitsablauf entsprechen;
ein Verarbeitungsmodul (156) zum Verarbeiten der Bilder und zum Konfigurieren der Ultraschallbildgebungseinrichtung (110) mit dem ausgewählten Einrichtungseinstellparameter; und
ein Kommunikationsmodul (158) zum Kommunizieren der mit Anmerkungen versehenen Bilder durch Anzeigen der mit Anmerkungen versehenen Bilder, die jedem Schritt in dem klinischen Arbeitsablauf entsprechen, mindestens entweder neben dem Echtzeitbild oder damit verschmolzen; und Interagieren eines Klinikers mit dem mit Anmerkungen versehenen Bild (350), während der Kliniker diese Schritte durchläuft.

10. System nach Anspruch 9, wobei die Steuerungseinheit (150) dazu ausgelegt ist, dem Kliniker zu ermöglichen, einen Arbeitsablauf basierend auf mindestens den Patienteninformationen und dem klinischen Verfahren auszuwählen.

11. System nach Anspruch 9 oder Anspruch 10, wobei der ausgewählte vordefinierte Arbeitsablauf basierend auf der Entscheidung des Klinikers ermöglicht, die Einstellparameter der Bildgebungseinrichtung (110) auszuwählen.

## Revendications

1. Procédé de guidage d'un clinicien réalisé par un système d'imagerie ultrasonore possédant une interface utilisateur, une unité de commande, une mémoire et un dispositif d'affichage d'image comprenant les étapes consistant à :
obtenir des informations patient (210) avec une procédure clinique pertinente par l'intermédiaire d'une interface utilisateur ;
sélectionner un flux de travail prédéfini et au moins un paramètre de réglage (220) de dispositif dans une bibliothèque d'informations, sur la base d'une décision de clinicien obtenue par l'intermédiaire de l'interface utilisateur ;
communiquer le flux de travail (240) sélectionné comprenant les étapes dans le flux de travail au clinicien en temps réel à l'aide du dispositif d'affichage d'image, au début de la procédure clinique ; et
configurer le système d'imagerie (250) à l'aide du paramètre de réglage de dispositif sélectionné afin de réaliser le flux de travail sélectionné ;
dans lequel la communication du flux de travail sélectionné consiste en outre à :
annoter une image (330) sur la base des informations patient et/ou d'étapes du flux de travail, l'image comprenant des images d'échantillon et des images en temps réel du patient ;
afficher une image annotée (340), correspondant à chaque étape du flux de travail clinique, en dehors de l'image en temps réel et/ou mélangée dans celle-ci ; et
faire interagir un clinicien et l'image annotée (350).

2. Procédé selon la revendication 1, dans lequel la bibliothèque d'informations (230) possède des images d'échantillon annotées et des images patient en temps réel annotées sur la base des informations patient et des flux de travail prédéfinis.

3. Procédé selon l'une quelconque des revendications précédentes, dans lequel l'étape consistant à communiquer un flux de travail (240) prédéfini consiste à afficher le flux de travail étape par étape à mesure que le clinicien avance dans les diverses étapes de la procédure.

4. Procédé selon l'une quelconque des revendications précédentes, dans lequel chaque étape du flux de travail est affichée avec des images annotées correspondantes, les images annotées étant affichées en dehors des images ou mélangées à celles-ci.

5. Procédé selon la revendication 1, le procédé consistant en outre à : identifier au moins un paramètre de réglage de dispositif sur la base d'informations patient et du flux de travail sélectionné, le paramètre de réglage de dispositif étant identifié comme correspondant à chaque étape du flux de travail.

6. Procédé selon la revendication 5, le procédé consistant à : prérégler des paramètres de réglage de dispositif du système d'imagerie à l'aide des paramètres de réglage d'image identifiés, les paramètres de réglage d'image étant ajustés à chaque étape du flux de travail.

7. Procédé selon l'une quelconque des revendications 1 à 6, le procédé consistant en outre à : configurer automatiquement le système d'imagerie ultrasonore pendant que le clinicien avance dans les étapes du flux de travail.

8. Procédé selon l'une quelconque des revendications précédentes, dans lequel le flux de travail prédéfini sélectionné correspond à la procédure clinique de sorte que les paramètres de réglage de dispositif de système d'imagerie soient sélectionnés.

9. Système d'imagerie ultrasonore (100) comprenant :
un dispositif d'imagerie ultrasonore (110) possédant une sonde équipée d'un ensemble transducteur ;
un dispositif d'affichage d'image (120) associé au dispositif d'imagerie conçu pour communiquer visuellement avec un clinicien ;
une mémoire (140) conçue pour mémoriser divers flux de travail prédéfinis et des images annotées correspondant à diverses étapes du flux de travail ;
une interface utilisateur (130) conçue pour faciliter une interaction du clinicien avec le dispositif d'imagerie (110) et une interaction entre le dispositif d'imagerie (110) et le dispositif d'affichage (120) de sorte qu'un flux de travail prédéfini puisse être sélectionné ; et
une unité de commande (150) conçue pour guider le clinicien en affichant des étapes dans le flux de travail avec les images annotées correspondant à chaque étape, les images annotées étant basées sur des informations patient et/ou des étapes du flux de travail, l'image comprenant des images d'échantillon et des images en temps réel du patient ;
dans lequel l'unité de commande (150) comprend :
un module d'informations (152) conçu pour accéder aux flux de travail depuis la mémoire et les informations patient ;
un module de décision (154) permettant de sélectionner un flux de travail souhaité sur la base d'au moins une décision de clinicien obtenue par l'intermédiaire de l'interface utilisateur et de paramètres de réglage de dispositif correspondant à chaque étape du flux de travail ;
un module de traitement (156) permettant de traiter des images et de configurer le dispositif d'imagerie ultrasonore (110) avec le paramètre de réglage de dispositif sélectionné ; et
un module de communication (158) permettant de communiquer les images annotées en affichant les images annotées, correspondant à chaque étape du flux de travail clinique, en dehors de l'image en temps réel et/ou mélangées dans celle-ci ; et de faire interagir un clinicien avec l'image annotée (350) pendant que le clinicien avance dans ces étapes.

10. Système selon la revendication 9, dans lequel l'unité de commande (150) est conçue pour permettre que le clinicien sélectionne un flux de travail sur la base au moins d'informations patient et d'une procédure clinique.

11. Système selon la revendication 9 ou la revendication 10, dans lequel le flux de travail prédéfini sélectionné basé sur la décision clinique permet que les paramètres de réglage du dispositif d'imagerie (110) soient sélectionnés.
